(19) Europäisches Patentamt / European Patent Office / Office européen des brevets

(11) **EP 3 437 564 B1**

(12) **EUROPEAN PATENT SPECIFICATION**

(45) Date of publication and mention
of the grant of the patent:
**08.05.2024 Bulletin 2024/19**

(21) Application number: **17773894.5**

(22) Date of filing: **23.02.2017**

(51) International Patent Classification (IPC):
*A61B 8/12* (2006.01)    *H04R 17/00* (2006.01)
*B06B 1/06* (2006.01)    *A61B 8/00* (2006.01)

(52) Cooperative Patent Classification (CPC):
**A61B 8/12; A61B 8/4483; A61B 8/4494;
B06B 1/0633; B06B 1/0677; H04R 17/00**

(86) International application number:
**PCT/JP2017/006740**

(87) International publication number:
**WO 2017/169349 (05.10.2017 Gazette 2017/40)**

(54) **ULTRASOUND OSCILLATOR UNIT**

ULTRASCHALLOSZILLATOREINHEIT

UNITÉ D'OSCILLATEUR À ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.04.2016 JP 2016074547**

(43) Date of publication of application:
**06.02.2019 Bulletin 2019/06**

(73) Proprietor: **FUJIFILM Corporation
Tokyo 106-8620 (JP)**

(72) Inventors:
- **YAMAMOTO, Katsuya
  Ashigarakami-gun
  Kanagawa 258-8538 (JP)**

- **MORIMOTO, Yasuhiko
  Ashigarakami-gun
  Kanagawa 258-8538 (JP)**

(74) Representative: **Klunker IP
Patentanwälte PartG mbB
Destouchesstraße 68
80796 München (DE)**

(56) References cited:
JP-A- H0 879 895    JP-A- H04 119 800
JP-A- H10 282 074    JP-A- 2010 022 931
US-A1- 2006 103 265    US-A1- 2009 088 647

## Description

## BACKGROUND OF THE INVENTION

1. Field of the Invention

The present invention relates to an ultrasonic oscillator unit having an ultrasonic oscillator wiring structure for realizing a small-sized ultrasonic device.

2. Description of the Related Art

Ultrasonic endoscopes are ones in which an ultrasonic observation part is provided at a distal end part of an endoscope with observation of the gallbladder or the pancreas by an alimentary canal as a main purpose. In order to safely insert the ultrasonic endoscope into the alimentary canal, an optical sensor, illumination means, an air supply port, a water supply port, and a suction port in addition to the ultrasonic observation part are provided at the distal end part of the ultrasonic endoscope, similarly to ordinary endoscopes that are not provided with the ultrasonic observation part. For that reason, the external diameter of the distal end part of the ultrasonic endoscope increases, and causes a decrease in the operability of the ultrasonic endoscope and an increase in the burden on a patient into which the distal end part of the ultrasonic endoscope is to be inserted.

Thus, in order to improve the operability of the ultrasonic endoscope and mitigate the burden on the patient, the ultrasonic observation part is required to be small-sized. Thus, in recent years, attempts have been made to improve the workability in wiring work and make the ultrasonic observation part of the ultrasonic endoscope small-sized are made (refer to JP4445764B, JP5399594B, JP1996-004359B (JP-H08-004359B), JP4980653B, and JP3802756B).

JP4445764B discloses an ultrasonic oscillator unit having an ultrasonic oscillator array that has an acoustic matching layer, piezoelectric elements, and a back surface damping layer; a rigid board electrically connected to the respective piezoelectric elements in the vicinity of a central part of the ultrasonic oscillator array in a width direction thereof; a signal cable bundle including a plurality of signal core wires; and a flexible printed wiring board that is interposed between the rigid board and the signal cable bundle to electrically connect both. Moreover, the ultrasonic oscillator array, and the cable bundle and the flexible printed wiring board are separate structures, both are connected to each other using thermocompression bonding as a means, and thereafter, the flexible printed wiring board is configured in a multiple-folded form.

JP5399594B discloses an ultrasonic endoscope having an ultrasonic transmission/reception unit that transmits and receives ultrasonic waves; a wiring board electrically connected to a back side of the ultrasonic transmission/reception unit; a plurality of driver wires electrically connected to the wiring board; and a housing that houses the wiring board to hold the ultrasonic transmission/reception unit. The wiring board has a rigid circuit board electrically connected to a plurality of ultrasonic oscillators in the vicinity of central parts thereof in a width direction; and an enveloping part that wraps and bundles the driver wires, and is inserted into a housing in a state where the driver wires are wrapped and bundled by the enveloping part.

JP1996-004359B (JP-H08-004359B) discloses an ultrasound probe in which signal lines are alternately connected from both sides of an ultrasonic oscillator array disposed on a convex surface and electrodes are led out from one side surface side by a single flexible printed wiring board having conductive paths formed on both surfaces thereof.

JP4980653B discloses an electronic scanning type ultrasonic probe having respective pad electrodes of a pad electrode group that are arranged on an oscillator board of an ultrasonic oscillator unit so as to extend from the vicinity of a central part of the ultrasonic oscillator array in a width direction thereof and that are electrically connected to ultrasonic oscillators; and a coaxial cable assembly having a comb-like lead electrode group. Upon connection between the pad electrodes of the ultrasonic oscillator unit and leads of the coaxial cable assembly, alignment between the respective pad electrodes and the comb-like lead electrode group is performed.

JP3802756B discloses an ultrasonic probe including a printed board having first and second signal pattern groups electrically connected to electrodes of an ultrasonic oscillator array in the vicinity of a central part of the ultrasonic oscillator array in a width direction thereof and electrically connected to halves of the electrodes of the ultrasonic oscillator array, respectively. The first and second signal pattern groups are wired with the coaxial cable in different directions, respectively.

US 2006/103265 A1 discloses an ultrasonic transducer array comprising a backing material having a curved surface and plural ultrasonic transducers arranged on the curved surface of said backing material in a manner of one of directly and indirectly. Each of said plural ultrasonic transducers includes a first conducting material layer, a piezoelectric material layer and a second conducting material layer. A surface of said piezoelectric material layer at an opposite side to said backing material has an area larger than that of another surface of said piezoelectric material layer at a side of the backing material. In one form, a backing material of an ultrasonic transducer array has a half columnar shape.

JPH0879895A discloses a convex ultrasonic probe. An array arrangement face curved in a projection way is formed to the surface of a backing material. Furthermore, plural wiring circuits to be connected to electrodes of a piezoelectric element are formed to circuit arrangement faces of both side faces of the backing material. One terminal of the circuit reaches a lower ridge and its upper end reaches the array arrangement face in excess of an upper ridge.

## SUMMARY OF THE INVENTION

Meanwhile, normally, in the ultrasonic observation part provided at the distal end part of the ultrasonic endoscope, the ultrasonic oscillators are disposed in an array, and the cables are wired to the ultrasonic oscillators, respectively. However, since the external diameter of the ultrasonic observation part is small and the wiring within the ultrasonic observation part is a complicated task, the wiring is manually performed in many cases. Hence, since it is necessary to wire the cables in high density within the ultrasonic observation part in addition to the handling of the cables within the ultrasonic observation part with a small external diameter being complicated, this becomes a cause that the workability is poor and the manufacturing costs of the ultrasonic endoscope become high. In this way, there is a problem that size reduction of the ultrasonic observation part is very difficult, from viewpoints of the manufacture stability of the ultrasonic observation part and the manufacturing costs.

Additionally, in the techniques disclosed in JP4445764B and JP1996-004359B (JP-H08-004359B), a structure in which the flexible printed wiring board of the ultrasonic oscillator unit is folded up is provided. Therefore, there is a problem that the wiring structure of the cable bundle and the flexible printed wiring board is complicated. Even though the ultrasonic oscillator array, the cable bundle, and the flexible printed wiring board are connected to each other by thermocompression bonding, there is still a problem in the workability of wiring. Particularly, in JP4445764B, there are problems that, during the manufacture of the ultrasonic oscillator unit, a burden is applied on a cable in a case where the flexible printed wiring board is folded up multiple times, and the cable wiring line to which the burden is applied is disconnected.

Additionally, in JP4445764B, JP5399594B, JP4980653B, and JP3802756B the electrodes of the ultrasonic oscillator array and the wiring board are electrically connected to each other in the vicinity of the central part of the ultrasonic oscillator array in the width direction thereof. In this structure, there are problems that the manufacture is significantly difficult and the success rate of the manufacture is not high.

The invention has been made in order to solve such related-art problems, and an object thereof is to provide an ultrasonic oscillator unit that can be small-sized and has a wiring structure with excellent workability in a case where wiring is performed.

In order to achieve the above object, the invention provides an ultrasonic oscillator unit according to claim 1 of the appended claims.

Further aspects are specified in the dependent claims.

In the invention, the cable wiring part is provided along the backing material layer that becomes thinner toward the side opposite to the arrangement surface of the ultrasonic oscillator.

Accordingly, according to the invention, since the space for wiring the cables in the ultrasonic oscillator array can be secured, the workability during wiring can be enhanced, and the ultrasonic oscillator unit can be small-sized using a simple structure.

## BRIEF DESCRIPTION OF THE DRAWINGS

Fig. 1 is a schematic configuration view illustrating an example of the configuration of an ultrasonic inspection system using an ultrasonic endoscope to which an ultrasonic oscillator unit of the invention is applied.
Fig. 2 is a partially enlarged plan view illustrating an endoscope distal end part of the ultrasonic endoscope illustrated in Fig. 1.
Fig. 3 is a view of the endoscope distal end part taken along line I-I illustrated in Fig. 2 and seen from an arrow direction and is a partially cross-sectional view of the endoscope distal end part of the ultrasonic endoscope illustrated in Fig. 2.
Fig. 4 is a view of the endoscope distal end part taken along line II-II illustrated in Fig. 3 and seen from an arrow direction and is a cross-sectional view of an example of an ultrasonic observation part of the endoscope distal end part of the ultrasonic endoscope illustrated in Fig. 3.
Fig. 5 is a longitudinal cross-sectional view of the endoscope distal end part of the ultrasonic endoscope of Embodiment 2 cut in a longitudinal direction thereof so as to pass through the center of the endoscope distal end part in a width direction thereof.
Fig. 6 is a view of the endoscope distal end part taken along line III-III illustrated in Fig. 5 and seen from an arrow direction and is a cross-sectional view of an example of the ultrasonic observation part of the endoscope distal end part of the ultrasonic endoscope illustrated in Fig. 5.

## DESCRIPTION OF THE PREFERRED EMBODIMENTS

Hereinafter, ultrasonic oscillator units of the invention will be described in detail on the basis of preferred embodiments illustrated in the attached drawings.

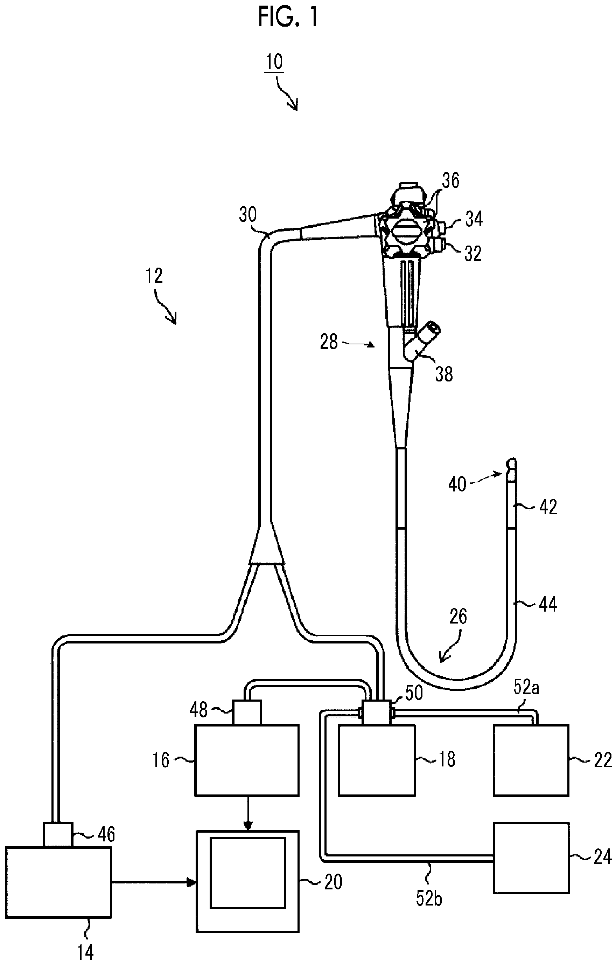

A schematic configuration view illustrating an example of the configuration of an ultrasonic inspection system using an ultrasonic endoscope to which an ultrasonic oscillator unit of the invention is applied is illustrated in Fig. 1.

An ultrasonic inspection system 10 has an ultrasonic endoscope 12 that is disposed at the distal end of the ultrasonic inspection system 10 and images the inside of a body cavity of a subject, an ultrasonic wave processor device 14 that creates an ultrasound image, an endoscope processor device 16 that creates an endoscopic image, a light source device 18 that supplies the illumination light for illuminating the inside of the body cavity of the subject to the ultrasonic endoscope 12 via a light guide (not illustrated), and a monitor 20 that displays the ultrasound image and the endoscopic image that are acquired from the ultrasonic wave processor device 14 and the endoscope processor device 16.

The ultrasonic inspection system 10 further has a water supply pump with that is stored in the light source device 18 and that supplies water to the ultrasonic endoscope 12 (not illustrated), a water supply tank 22 that stores the water to be supplied to the ultrasonic endoscope 12 using the water supply pump, an air supply pump (not illustrated) that is stored in the light source device 18 for supplying air to the ultrasonic endoscope 12, and a suction pump 24 for suctioning an observation target from the endoscope distal end part 40 of the ultrasonic endoscope 12 to be described below.

The ultrasonic wave processor device 14, the endoscope processor device 16, the light source device 18, the water supply tank 22, the suction pump 24, the water supply pump, and the air supply pump are connected to the ultrasonic endoscope 12, using a universal cord 30 (to be described below) of the ultrasonic endoscope 12.

The ultrasonic endoscope 12 of the ultrasonic inspection system 10 includes an insertion part 26 that has a distal end side inserted into the body cavity of the subject in order to observe targets, such as the gallbladder and the pancreas, and is inserted into the body cavity of the subject, which is disposed on a distal end side of the ultrasonic endoscope 12, an operating part 28 that is provided continuously with a proximal end part of the insertion part 26 for allowing operators, such as a doctor and an engineer, to perform an operation, and the universal cord 30 that has one end connected to the operating part 28 and the other end connected to a plurality of devices for controlling the ultrasonic endoscope 12.

The ultrasonic wave processor device 14 of the ultrasonic inspection system 10 is a device for creating and supplying ultrasonic signals (data) for creating the ultrasonic waves in an ultrasonic oscillator array 100 of an ultrasonic oscillator unit 68 of an ultrasonic observation part 58 of an endoscope distal end part 40 of the insertion part 26 of the ultrasonic endoscope 12 to be described below. Additionally, the ultrasonic wave processor device 14 is a device for receiving and acquiring the echo signals (data), which is reflected from the region to be observed to which the ultrasonic waves are radiated, with the ultrasonic oscillator array 100, and for creating the ultrasound image that is obtained by performing various kinds of signal (data) processing on the acquired echo signals and is displayed on the monitor 20.

The endoscope processor device 16 of the of the ultrasonic inspection system 10 is a device for receiving and acquiring captured image signals (data) acquired from the region to be observed illuminated with the illumination light from the light source device 18 in the endoscope observation part 56 of the endoscope distal end part 40 of the insertion part 26 of the ultrasonic endoscope 12 to be described below and for creating the endoscopic image that is obtained by performing various kinds of signal (data) processing and image processing on the acquired image signals and is displayed on the monitor 20.

In addition, the processor devices 14 and 16 may be constituted of processors, such as a personal computer (PC).

In order to image the region to be observed within the body cavity to acquire the image signals with the endoscope observation part 56 of the ultrasonic endoscope 12 to be described below, the light source device 18 is a device for generating Illumination light, such as white light consisting of three primary color lights, such as red light (R), green light (G), and blue light (B), or specific wavelength light to supply the Illumination light to the ultrasonic endoscope 12 to propagate the illumination light with a light guide or the like within the ultrasonic endoscope 12 (not illustrated), and emitting the illumination light from the endoscope observation part 56 of the distal end part 40 of the insertion part 26 of the ultrasonic endoscope 12 for illuminating the region to be observed within the body cavity with the illumination light.

The monitor 20 of the ultrasonic inspection system 10 receives respective video signals created by the ultrasonic wave processor device 14 and the endoscope processor device 16 to display the ultrasound image and the endoscopic image. The monitor 20 is capable of appropriately displaying only any one image of the ultrasound image and the endoscopic image through switching and simultaneously displaying both the images. In addition, a monitor for displaying the ultrasound image and a monitor for displaying the endoscopic image may be separately provided, or the ultrasound image and the endoscopic image may be displayed using other arbitrary forms.

The operating part 28 of the ultrasonic endoscope 12 has an air/water supply button 32 that is a switch for supplying air or supplying water to the endoscope distal end part 40 of the insertion part 26 to be described below, and a suction button 34 that is a switch that is disposed side by side with the air/water supply button 32 on the endoscope distal end part 40 side in a longitudinal direction of the ultrasonic endoscope 12 for suctioning the observation target while puncturing the observation target at a distal end of a puncturing needle of a treatment tool (not illustrated) delivered from the endoscope distal end part 40.

Moreover, the operating part 28 of the ultrasonic endoscope 12 has angle knobs 36 that are a pair of knobs each disposed on each side surface of the operating part 28 so as to sandwich the air/water supply button 32 and the suction button 34 and freely bend a bending part 42 (to be described below) vertically and horizontally by rotationally moving the respective knobs, and a treatment tool insertion port (forceps port) 38 that is disposed between the air/water supply button 32 and the insertion part 26 and allows treatment tools, such as forceps, a puncturing needle, and a high-frequency knife, which are delivered from the endoscope distal end part 40, to be inserted therethrough.

The insertion part 26 of the ultrasonic endoscope 12 has the endoscope distal end part (distal end rigid part) 40 that has ultrasonic oscillators 98 of the ultrasonic oscillator unit 68, an observation window 76 of an imaging unit 64, and the like (to be described below) and is formed of a rigid member, the bending part 42 that is provided continuously with a proximal end side of the endoscope distal end part 40 and is freely bendable, and a flexible part 44 that couples a proximal end sides of the bending part 42 and a distal end side of the operating part 28 to each other, and is thin, elongated, and flexible, sequentially from the distal end side.

The universal cord 30 of the ultrasonic endoscope 12 is a cord for connecting a plurality of devices for controlling the ultrasonic endoscope 12 and the ultrasonic endoscope 12 to each other, and is provided at a rear end of the ultrasonic endoscope 12. An ultrasonic wave connector 46 connected to the ultrasonic wave processor device 14, an endoscope connector 48 connected to the endoscope processor device 16, and a light source connector 50 connected to the light source device 18, the water supply tank 22, the suction pump 24, the water supply pump (not illustrated), and the air supply pump (not illustrated) are attachably and detachably connected to the other end part of the universal cord 30 with respect to a distal end part of the ultrasonic endoscope 12. Additionally, an air/water supply tube 52a having the other end connected to the water supply tank 22, and a suctioning tube 52b having the other end connected to the suction pump 24 are connected to the light source connector 50.

The air/water supply button 32 of the operating part 28 is a switch that controls the supply of air or water to the endoscope distal end part 40 of the insertion part 26, is connected the other end of a pipe line (not illustrated), which passes through the inside of the ultrasonic endoscope 12 and has one end leading to an air/water supply nozzle 62 of the endoscope observation part 56 of the endoscope distal end part 40 to be described above, and is connected to the other end of a pipe line (not illustrated) that has one end leading to the water supply tank 22 and the water supply pump (not illustrated) or the air supply pump (not illustrated). By pushing the air/water supply button 32, the water supply pump or the air supply pump, and the pipe lines communicating with the air/water supply nozzle 62 and the water supply tank 22 are connected to each other, and the water or air stored in the water supply tank 22 is supplied to the air/water supply nozzle 62. In addition, well-known methods, such as configuring the air/water supply button 32 with a two-step switching type, can be appropriately used as methods for switching connections of pipe lines of the water supply pump and the air supply pump.

The suction button 34 of the operating part 28 is a switch that controls the suction operation in the endoscope distal end part 40 of the insertion part 26, is connected the other end of a treatment tool insertion channel 61 that passes through the inside of the ultrasonic endoscope 12 and has one end leading to the treatment tool delivery port 60 of the endoscope distal end part 40, and is connected to the other end of a pipe line (not illustrated) that has one end leading to the suction pump 24. As for the suction button 34, by pushing the suction button 34 similarly to the above-described air/water supply button 32, the pipe lines leading to the treatment tool insertion channel 61 and the suction pump 24 are connected to each other, and suction is performed from the treatment tool delivery port 60. Additionally, in a case where a treatment tool (not illustrated) having a puncturing needle is inserted through the treatment tool insertion channel 61, suction of tissue of the observation target is performed from the distal end of the puncturing needle by pushing the suction button 34.

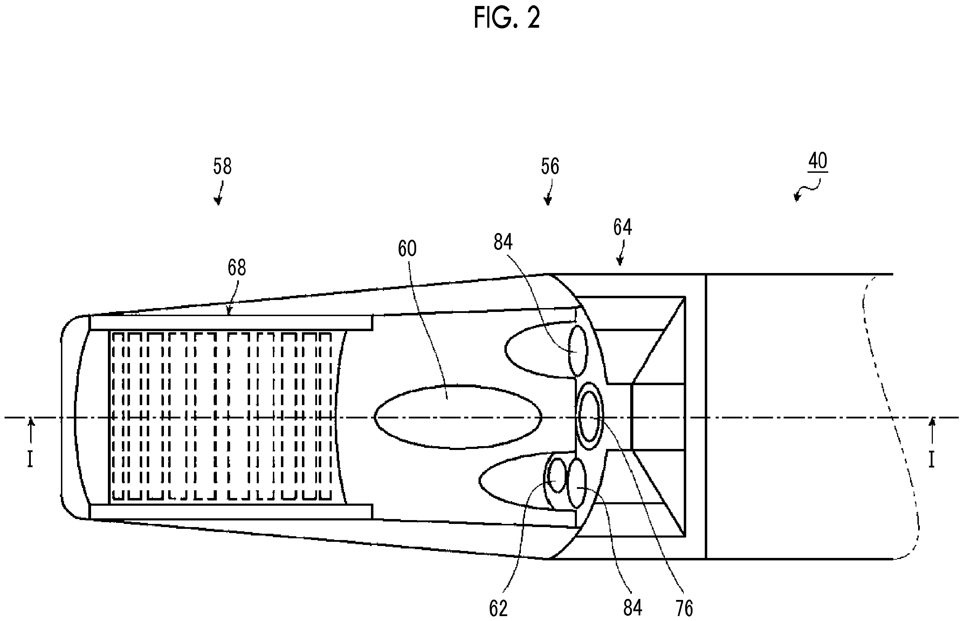

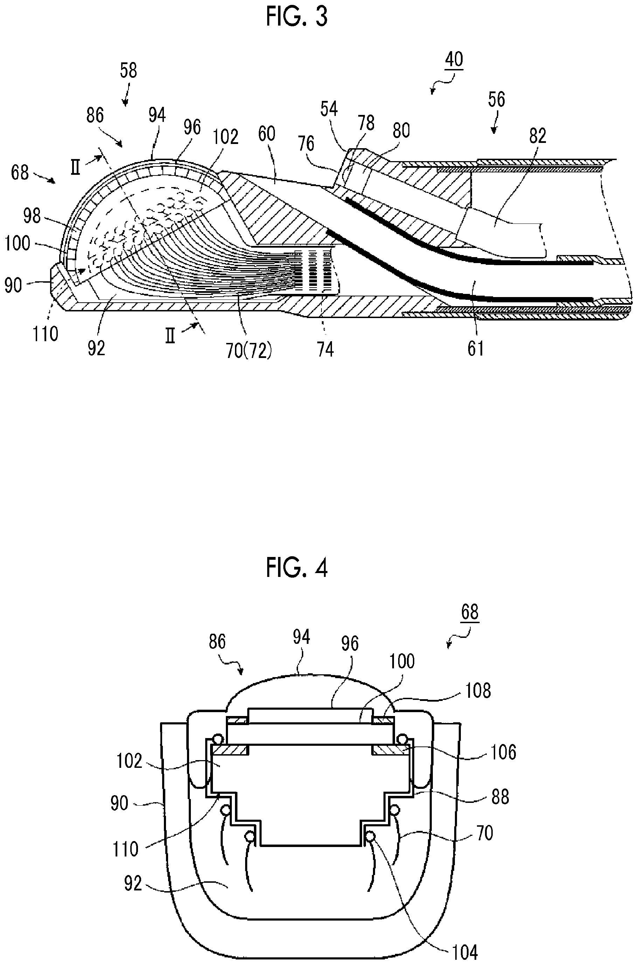

A partially enlarged plan view illustrating the endoscope distal end part of the ultrasonic endoscope illustrated in Fig. 1 is illustrated in Fig. 2. Additionally, a view of the endoscope distal end part taken along line I-I illustrated in Fig. 2 and seen from an arrow direction and a partially cross-sectional view of the endoscope distal end part of the ultrasonic endoscope illustrated in Fig. 2 are illustrated in Fig. 3. As illustrated in Figs. 2 and 3, the endoscope distal end part 40 of the insertion part 26 has an inclined surface part 54 formed on a proximal end side thereof, the inclined surface part 54 being an inclined surface having a large angle of elevation in a direction of a proximal end of the endoscope distal end part 40 with respect to a bottom surface of the endoscope distal end part 40, and has the endoscope observation part 56 that is provided on the inclined surface part 54 for acquiring the endoscopic image, the ultrasonic observation part 58 that is provided on the distal end side of the endoscope distal end part 40 for acquiring the ultrasound image, the treatment tool delivery port 60 that is provided between the endoscope observation part 56 and the ultrasonic observation part 58 and that delivers a treatment tool (not illustrated) within the body cavity of the subject, the treatment tool insertion channel 61 that allows the treatment tool insertion port 38 and the treatment tool delivery port 60 of the operating part 28 to communicate with each other for allowing the treatment tool to be inserted therethrough, and the air/water supply nozzle 62 that is provided between the endoscope observation part 56 and the treatment tool delivery port 60 for washing foreign matter or the like adhering to the endoscope observation part 56.

The bending part 42 of the insertion part 26 is formed by coupling a plurality of bendable pieces to each other and is provided continuously with the proximal end side of the endoscope distal end part 40. Additionally, the bending part 42 is freely bendable vertically and horizontally by the rotational movement of the pair of angle knobs 36 provided at the operating part 28. In this way, since the bending part 42 is remotely and freely bending-operated by using the angle knobs 36 as operating means, the endoscope distal end part 40 can be directed to a direction desired to an operator.

Since the flexible part 44 of the insertion part 26 couples a proximal end side of the bending part 42 and the distal end side of the operating part 28 to each other, and is thin, elongated, and flexible, even in a case where the flexible part 44 is within the body cavity of the subject having a complicated structure, the flexible part 44 can be inserted so as to follow the bending-operated bending part 42.

The endoscope observation part 56 of the endoscope distal end part 40 has the imaging unit 64 that is provided so as to pass through the inside of the ultrasonic endoscope 12 from the center of the inclined surface part 54 and captures the endoscopic image, and an illumination unit 66 that is disposed side by side with the imaging unit 64 for illuminating a region to be observed using the illumination light from the light source device 18.

Fig. 4 is a view of the endoscope distal end part taken along line II-II illustrated in Fig. 3 and seen from an arrow direction and is a cross-sectional view of an example of the ultrasonic observation part of the distal end part of the ultrasonic endoscope illustrated in Fig. 3. The ultrasonic observation part 58 of the endoscope distal end part 40 to be inserted into the inside of the body of the subject in order to observe targets, such as the gallbladder and the pancreas has a cable dispersion part 72 including a plurality of cables 70 electrically connecting the ultrasonic oscillator unit 68, which transmits and receives the ultrasonic signals with respect to the observation target, to the universal cord 30 that transmit driving signals of the ultrasonic waves to the ultrasonic oscillator unit 68, the universal cord 30 being connected to the ultrasonic wave processor device 14 that analyzes signals of the reflected waves from the observation target that are received from the ultrasonic oscillator unit 68, and creates the ultrasound image, and a cable covering part 74 that binds the cable dispersion part 72.

In addition, since Fig. 4 is a schematic view illustrated for description of the invention and does not illustrate the details, it is needless to say that the arrangement locations, sizes, and shapes of the respective members illustrated in Fig. 4 may be appropriately changed without departing from the scope of the invention.

The treatment tool delivery port 60 of the endoscope distal end part 40 is provided on a distal end side of the imaging unit 64, and a treatment tool (not illustrated), which is inserted from the treatment tool insertion port 38 of the operating part 28 and passes through the treatment tool insertion channel 61, is delivered from the treatment tool delivery port 60.

Additionally, the air/water supply nozzle 62 of the endoscope distal end part 40 is a nozzle that is provided between the imaging unit 64 and the treatment tool delivery port 60 for washing the observation window 76 of the imaging unit 64 to be described below. By pushing the air/water supply button 32 of the operating part 28, air or water is supplied from the air supply pump (not illustrated) or the water supply pump (not illustrated) through a flow channel (not illustrated) for air supply or water supply provided within the ultrasonic endoscope 12 to the air/water supply nozzle 62.

Additionally, the cable dispersion part 72 of the endoscope distal end part 40 includes the plurality of cables 70 electrically connected to the ultrasonic oscillator unit 68, and is a portion in which the plurality of cables 70 are not bound in the cable covering part 74. Since the cable dispersion part 72 I fixed using electrical connection means, such as soldering or conductive paste in a wiring portion between the ultrasonic oscillator unit 68 and the cables 70, the cable dispersion part 72 is weak to a mechanical external force and disconnection thereof is likely to occur. For that reason, the cable dispersion part 72 is fixed with a filler layer 92 of the ultrasonic oscillator unit 68 (to be described below) in order to prevent the disconnection.

The imaging unit 64 of the endoscope observation part 56 has a transparent observation window 76 disposed at the inclined surface part 54 for protecting an imaging optical system disposed at the rear thereof, an objective lens 78 of an observation optical system that is disposed behind the observation window 76 and inside the endoscope distal end part 40, an imaging element 80, such as a charge coupled device (CCD) or a complementary metal-oxide semiconductor (CMOS) that is disposed at a focusing position of the objective lens 78 inside the endoscope distal end part 40, and a signal cable 82 electrically connected to the imaging element 80, and the universal cord 30 connected to the light source device 18 through a pipe line (not illustrated) inside the ultrasonic endoscope 12.

The illumination unit 66 of the endoscope observation part 56 has a pair of transparent illumination windows 84 that are provided side by side at the imaging unit 64, are provided side by side on both sides of the observation window 76 in the inclined surface part 54 and protect an illumination optical system disposed at rear thereof, and the light guide (not illustrated) that is disposed behind the illumination windows 84 and inside the endoscope distal end part 40 for transmitting the illumination light from the light source device 18 to the illumination windows 84.

The observation window 76 of the imaging unit 64 is disposed at the inclined surface part 54, and the image light of the observation target incident from the observation window 76 is focused on an imaging surface of the imaging element 80 by the objective lens 78. The imaging element 80 photoelectrically converts the image light of the observation target transmitted through the observation window 76 and the objective lens 78 and focused on the imaging surface of the imaging element 80 and outputs imaging signals to the endoscope processor device 16. The imaging signals output from the imaging element 80 are transmitted to the endoscope processor device 16 via the signal cable 82 and the universal cord 30, and the endoscope processor device 16 performs signal processing and image processing on the transmitted imaging signals and then displays the processed signals on the monitor 20 as an endoscopic optical image.

The illumination windows 84 of the illumination unit 66 are a pair of windows that are provided side by side on both sides of the observation window 76 of the imaging unit 64, and an exit end of the light guide (not illustrated), which guides the illumination light from the light source device 18, to the observation window 76, is connected to the illumination windows 84. The light guide extends from the illumination windows 84 to the light source device 18 through the inside of the ultrasonic endoscope 12, and an incident end of the light guide is stored within the light source device 18. The Illumination light emitted by the light source device 18 is transmitted to the light guide and is radiated from the illumination windows 84 to the observation target.

The ultrasonic oscillator unit 68 of the ultrasonic observation part 58 has a laminated body 86 that is disposed at an upper part of a distal end side of the ultrasonic observation part 58 and transmits and receives the ultrasonic waves, and has a laminated structure, a cable wiring part 88 that are electrically connected to the laminated body 86 and the cable dispersion part 72 including the plurality of cables 70, a housing 90 that surrounds the side surfaces and lower surface of the laminated body 86 and the cable wiring part 88 in order to protect from the outside of the ultrasonic observation part 58, and the filler layer 92 that fills a gap between the laminated body 86 and the housing 90 in order to fix the wiring portion of the cable wiring part 88.

The laminated body 86 of the ultrasonic oscillator unit 68 has a laminated structure, and has an acoustic lens 94 that is located at an uppermost part for converging the ultrasonic waves output from the ultrasonic oscillator array 100 to be described below and the ultrasonic waves reflected from the observation target, an acoustic matching layer 96 that is located under the acoustic lens 94 for matching the acoustic impedance of the ultrasonic oscillators 98 constituting the ultrasonic oscillator array 100 with the acoustic impedance of the observation target, the ultrasonic oscillator array 100 which is located under the acoustic matching layer 96 and in which the plurality of elongated ultrasonic oscillators 98 that transmit and receive the ultrasonic waves are aligned in an array, and a backing material layer 102 that is located under the ultrasonic oscillator array 100 for mechanically supporting the ultrasonic oscillator array 100 and damping the ultrasonic waves propagated to a lower side of the ultrasonic oscillator array 100. As illustrated in Figs. 3 and 4, the acoustic matching layer 96 and the ultrasonic oscillator array 100 are disposed in a semicylindrical shape, and the acoustic lens 94 is disposed along the acoustic matching layer 96 disposed in the semicylindrical shape. Additionally, in the cross-sectional view illustrated in Fig. 3, the backing material layer 102 has a semicircular columnar shape.

The cable wiring part 88 of the ultrasonic oscillator unit 68 has wiring pad rows 104 that are electrically connected to the electrode part 106 of the ultrasonic oscillator array 100 to be described below and the plurality of cables 70 constituting the cable dispersion part 72, are disposed along a stepwise stepped part 110 formed on each side surface of the backing material layer 102 (to be described below) in a width direction thereof, and have a plurality of electrode pads aligned in a region corresponding to respective steps of the stepped part 110. That is, the plurality of wiring pad rows 104 of the cable wiring part 88 are aligned in a stepwise shape from a lower end along the stepped part 110 of the backing material layer 102. Additionally, although the cable wiring part 88 may be configured using a rigid wiring board, it is preferable that the cable wiring part 88 is configured using flexible wiring means, such as a flexible printed wiring board from a viewpoint of easiness of installation.

The housing 90 of the ultrasonic oscillator unit 68 is made of rigid members, such as hard resin, and protects the side surfaces and lower surface of the laminated body 86 and the cable wiring part 88 from the outside, and is disposed so as to abut against the side surfaces of the acoustic lens 94 of the laminated body 86 (to be described below) in the width direction of the laminated body 86 and surround the side surfaces and a lower part of the laminated body 86 in the width direction thereof and the cable wiring part 88.

The filler layer 92 of the ultrasonic oscillator unit 68 is provided so as to fill a gap between the housing 90, and the laminated body 86, particularly, the backing material layer 102 and the cable wiring part 88, and fixes a wiring portion between the cable wiring part 88 and the cables 70 to prevent disconnection of the wiring portion.

Moreover, it is preferable that the acoustic impedances of the filler layer 92 of the ultrasonic oscillator unit 68 and the backing material layer 102 are matched with each other such that the ultrasonic waves, which are oscillated from the ultrasonic oscillator array 100 of the laminated body 86 and propagated to a lower side thereof, are not reflected at a boundary between the filler layer 92 and the backing material layer 102 and such that the ultrasonic waves oscillated from the ultrasonic oscillator array 100 is reflected in the observation target or its peripheral part and sufficiently damp the ultrasonic waves propagated to the lower side of the ultrasonic oscillator array 100. For that reason, in a case where the acoustic impedance of the filler layer 92 is defined as Zp and the acoustic impedance of the backing material layer 102 is defined as Zb, it is preferable that an acoustic impedance reflectivity Q of the filler layer 92 and the backing material layer 102 expressed by the following Equation (1) is 50% or less.

$$Q = 100 \times |Zp - Zb|/(Zp + Zb) \qquad (1)$$

Here, the unit of the acoustic impedance Zp and Zb is $kg/m^2s$. In addition, kg represents kilogram, m represents meter and s represents second.

Additionally, in order for the acoustic impedance reflectivity Q of the filler layer 92 and the backing material layer 102 to be 50% or less, for example, a filler of the same material as the backing material layer 102 may be used for the material of the filler layer 92. In a case where hard rubber or the like to which an ultrasonic damping material, such as ferrite or ceramics, is added as the material of the backing material layer 102 is used, epoxy resin to which a heat conduction member, such as ceramics, is added may be used as the filler layer 92.

The above acoustic impedance reflectivity Q is an index showing the easiness of reflection of the ultrasonic waves (sound beams) on a boundary surface between the filler layer 92 and the backing material layer 102, that is, shows that the acoustic impedance of the filler layer 92 and the acoustic impedance of the backing material layer 102 are matched with each other as the value thereof is closer to 0%. In a case where the above acoustic impedance reflectivity is about 50% or less, the noise caused by the ultrasonic waves propagated to the lower side of the ultrasonic oscillator array 100 can be processed to such a degree that no hindrance is caused in the creation of the ultrasound image in the ultrasonic wave processor device 14 using the ultrasonic signals received in the ultrasonic oscillator array 100.

Additionally, in a case where the ultrasonic waves are oscillated from the ultrasonic oscillator array 100 of laminated body 86 of the ultrasonic oscillator unit 68, the driving signals transmitted from the ultrasonic wave processor device 14 to the ultrasonic oscillator array 100 become thermal energy and the ultrasonic oscillator array 100 generates heat. Therefore, it is preferable that the filler layer 92 has heat dissipation. For that reason, it is preferable that the thermal conductivity of the filler layer 92 is more than 1.0 W/(m·K), for example, the epoxy resin with to which the heat conduction member, such as ceramics, is added may be used as the filler layer 92. Here, W represents watt, m represents meter and K represents Kelvin.

The acoustic lens 94 of the laminated body 86 is a lens for converging the ultrasonic waves, and Is disposed so as to abut against the upper surfaces or side surfaces of the acoustic matching layer 96, the ultrasonic oscillator array 100, and the backing material layer 102, respectively, and cover the middle of each side surface of the backing material layer 102 in the width direction, in order to protect the acoustic matching layer 96, the ultrasonic oscillator array 100, and the backing material layer 102 that are laminated under the acoustic lens 94. Additionally, the acoustic lens 94 has a convex shape such that the acoustic lens 94 covers an upper part of the ultrasonic oscillator array 100 in the width direction of the laminated body 86 in order to converge the ultrasonic waves oscillated from the ultrasonic oscillator array 100 toward the observation target or in order to converge the ultrasonic waves reflected from the observation target toward the ultrasonic oscillator array 100. In addition, the acoustic lens 94 is disposed at the above-described position after the wiring task of the electrode part 106 and the upper electrode part 108 of the ultrasonic oscillator array 100 to be described below is completed. Additionally, the acoustic lens 94 is made of, for example, silicon-based resin, such as millable type silicone rubber or liquid silicone rubber, butadiene-based resin, polyurethane-based resin, or the like. Moreover, in order to match the acoustic impedance of the subject that is the observation target for ultrasonic observation with the acoustic impedance of the ultrasonic oscillators 98 that constitutes the ultrasonic oscillator array 100 and increase the transmittance of the ultrasonic waves to the subject, powder, such as titanium oxide, alumina, or silica, is mixed with the acoustic lens 94 as needed.

The acoustic matching layer 96 of the laminated body 86 is a layer for matching the acoustic impedances of the ultrasonic oscillator array 100 and the observation target, which are made of epoxy resin or the like, with each other. Since the acoustic matching layer 96 is installed such that a lower surface of the acoustic matching layer 96 is installed so as to abut against an upper surface of the ultrasonic oscillator array 100, but has a width shorter than the ultrasonic oscillator array 100 in the width direction of the laminated body 86, the acoustic matching layer 96 partially covers the upper surface of the ultrasonic oscillator array 100 such that both end parts or any one end part of the ultrasonic oscillator array 100 in the width direction thereof is removed. For that reason, the ultrasonic waves contributing to the observation of the target among the ultrasonic waves transmitted from the ultrasonic oscillator array 100 are only the ultrasonic waves that have passed through the acoustic matching layer 96, that is, only the ultrasonic waves transmitted from the ultrasonic oscillator array 100 in a region inside the side surfaces of the acoustic matching layer 96 in the width direction thereof.

The ultrasonic oscillator array 100 of the laminated body 86 is an array in which the ultrasonic oscillators 98 are aligned in a semicylindrical shape, transmit the ultrasonic signals to the observation target, and receive the ultrasonic waves reflected from the observation target to convert the received ultrasonic waves into electrical signals, is disposed such that the lower surface of the ultrasonic oscillator array 100 abuts against an upper surface of the backing material layer 102. The ultrasonic oscillator array 100 has the electrode part 106 electrically connected to the plurality of ultrasonic oscillators 98 and the cable wiring part 88 on a lower side of each side surface of the ultrasonic oscillator array 100 that are perpendicular to the arrangement surface of the plurality of ultrasonic oscillators 98 that constitute the ultrasonic oscillator array 100, and has the upper electrode part 108 electrically connected to the plurality of ultrasonic oscillators 98 constituting the ultrasonic oscillator array 100, and a grounding electrode (not illustrated) provided within the ultrasonic endoscope 12, on the upper surface of the ultrasonic oscillator array 100, and a surface thereof that is not covered with the acoustic matching layer 96 and is covered only with the acoustic lens 94.

Here, in a case where a position where the electrode part 106 is disposed is such that the workability in a case where wiring between the cable wiring part 88 and the electrode part 106 is performed is not impaired, the position does not need to be the lower side of each side surface of the ultrasonic oscillator array 100 that is strictly perpendicular to the arrangement surface of the plurality of ultrasonic oscillators 98. For that reason, in the invention, the expression "perpendicular to the arrangement surface of the plurality of ultrasonic oscillators 98 that constitute the ultrasonic oscillator array 100" means perpendicular or substantially perpendicular with an accuracy within a range of minus 5 degrees to plus 5 degrees with respect to a normal line of the arrangement surface of the plurality of ultrasonic oscillators 98.

In addition, the plurality of ultrasonic oscillators 98 constituting the ultrasonic oscillator array 100 are constituted of piezoelectric elements, and well-known piezoelectric elements made of, for example, lead zirconium titanate or polyvinylidene fluoride, can be used. Additionally, as methods of electrical connection between the electrode part 106 the ultrasonic oscillator array 100 and the cable wiring part 88 and between the upper electrode part 108 and wiring lines, well-known methods, such as methods using wire bonding, soldering, heat welding, a anisotropic conductive sheet, and anisotropic conductive paste, can be used as long as the methods are methods that do not impair the workability of the wiring task.

The backing material layer 102 of the laminated body 86, which is a feature of the invention, mechanically supports the ultrasonic oscillator array 100, suppresses the oscillation of the ultrasonic oscillator array 100, and damps the ultrasonic waves propagated to the lower side of the ultrasonic oscillator array 100, is disposed such that the upper surface of the backing material layer 102 abuts against the lower surface of the ultrasonic oscillator array 100, and has a width longer than the ultrasonic oscillator array 100 in the width direction of the laminated body 86. Additionally, the backing material layer 102 has the stepwise stepped part 110 that becomes thinner toward a side opposite to the arrangement surface of the plurality of ultrasonic oscillators 98 constituting the ultrasonic oscillator array 100, on each side surface of the backing material layer 102 in the width direction thereof, in order to widely secure a space required for the wiring between the electrode part 106 of the ultrasonic oscillator array 100 and the cables 70 of the cable dispersion part 72. In addition, the backing material layer 102 includes a material having rigidity, such as hard rubber, and an ultrasonic damping material made of, for example, ferrite or ceramics, is added as needed.

The electrode part 106, which is disposed on the lower side of each side surface of the ultrasonic oscillator array 100 perpendicular to the arrangement surface of the plurality of ultrasonic oscillators 98 that constitute the ultrasonic oscillator array 100, is electrically connected to an upper end of the cable wiring part 88, and is used to transmit the driving signals of the ultrasonic waves from the ultrasonic wave processor device 14 of the ultrasonic inspection system 10, to the ultrasonic oscillator array 100 via the cables 70 of the cable dispersion part 72, and to transmit the piezoelectric signals output after the ultrasonic oscillator array 100 receives the reflected ultrasonic waves, via the cables 70 to the ultrasonic wave processor device 14 that performs the analysis of the received ultrasonic signals and the creation of the ultrasound image. In this way, since the electrode part 106 is a structure disposed on each side surface of the ultrasonic oscillator array 100, wiring to the electrode part 106 can be relatively easily performed, and the success rate of manufacture of the laminated body 86. In addition, as methods of electrical connection between the cable wiring part 88 and the cables 70, well-known methods, such as methods using wire bonding, soldering, heat welding, a anisotropic conductive sheet, and anisotropic conductive paste, can be used as long as the methods are methods that do not impair the workability of the wiring task. Additionally, the electrode part 106 may be provided such that the cable wiring part 88 is disposed along each side surface of the backing material layer 102 in the width direction thereof, that is may be provided on an end surface side of the ultrasonic oscillator array 100 that becomes perpendicular to the arrangement surface of the plurality of ultrasonic oscillators 98, or may be connected to one end of the cable wiring part 88 and each side surface of the backing material layer 102 in the width direction thereof by being provided to extend to an upper end part of each side surface of the backing material layer 102 in the width direction thereof.

The upper electrode part 108, which is disposed on the surface that is an upper surface of the ultrasonic oscillator array 100, is not covered with the acoustic matching layer 96, and is covered only with the acoustic lens 94, is electrically connected to the grounding electrode (not illustrated) that includes one electrode pad connected to the plurality of respective ultrasonic oscillators 98 that constitute the ultrasonic oscillator array 100 and is provided within the ultrasonic endoscope 12, and is used to ground the driving signals for oscillating the ultrasonic waves, which are transmitted from the ultrasonic wave processor device 14 and transmitted from the electrode part 106 disposed on the lower side of each side surface of the ultrasonic oscillator array 100 to the respective ultrasonic oscillators 98 constituting the ultrasonic oscillator array 100, through the grounding electrode provided within the ultrasonic endoscope 12. Additionally, the upper electrode part 108 is disposed on the upper surface of the ultrasonic oscillator array 100. However, as described above, since the upper electrode part 108 is disposed in the region outside each side surface of the acoustic matching layer 96 in the width direction thereof, there is no particular influence on the transmission and reception of ultrasonic waves performed by the ultrasonic oscillator array 100. In addition, the upper electrode part 108 only has to be capable of grounding the plurality of respective ultrasonic oscillators 98 constituting the ultrasonic oscillator array 100, and it is needless to say that the upper electrode part 108 is not necessarily constituted of one electrode pad as long as the working efficiency of the wiring task in the upper electrode part 108 is not hindered.

The stepped part 110 formed on each side surface of the backing material layer 102 in the width direction thereof is provided in order to keep the plurality of cables 70, which are wired to the electrode part 106 in order to widely secure the space required for the wiring between the electrode part 106 and the cables 70, from overlapping each other. Since the backing material layer 102 has a function of damping the ultrasonic waves from the ultrasonic oscillator array 100 and the reflected wave from the observation target, which are propagated to the lower side of the ultrasonic oscillator array 100, it is preferable that the stepped part 110 is provided so as not to affect the output of the ultrasonic waves oscillated from the ultrasonic oscillator array 100. For this reason, it is preferable that the stepped part 110 is formed only in a region outside each side surface of the acoustic matching layer 96 in the width direction thereof on each side surface of the backing material layer 102 in the width direction thereof, in a region where the thickness from the surface on which the backing material layer 102 and the ultrasonic oscillator array 100 abut against each other is equal to or less than 3 mm. Additionally, the stepped part 110 may be formed in a region inside each side surface of the acoustic matching layer 96 in the width direction thereof on each side surface of the backing material layer 102 in the width direction thereof, in a region where the thickness from the surface on which the backing material layer 102 and the ultrasonic oscillator array 100 abut against each other exceeds 3 mm. In addition, the height of respective steps of the stepped part 110 and the width of the steps in the width direction of the backing material layer 102 may be appropriately changed to such a degree that the height and the width become smaller toward the side opposite to the arrangement surface of the plurality of ultrasonic oscillators 98 that constitute the ultrasonic oscillator array 100 and the workability in a case where the cables 70 of the cable dispersion part 72 are wired to the cable wiring part 88 disposed along the stepped part 110 is not impaired.

As described above, in the present embodiment, the wiring pad rows 104 of the cable wiring part 88 are aligned in a stepwise fashion along the stepped part 110 of the backing material layer 102 of the laminated body 86. Thus, the space for wiring the cables 70 of the cable dispersion part 72 and the cable wiring part 88 can sufficiently be secured, the plurality of cables 70 wired to the cable wiring part 88 do not overlap each other, the workability in the wiring task is improved. Additionally, since the wiring portion between the cables 70 and the cable wiring part 88 is filled with the filler layer 92, the risk that the cables 70 may be disconnected becomes low, for example, in a case where the ultrasonic endoscope 12 is manufactured and in a case where the ultrasonic endoscope 12 is used. Moreover, since the cable wiring part 88 connects the electrode part 106 of the ultrasonic oscillator array 100 and the end surface side of the ultrasonic oscillator array 100 in the width direction to each other, the wiring between the cable wiring part 88 and the electrode part 106 is relatively easy, and the success rate in a case where the ultrasonic oscillator unit 68 is manufactured can be improved.

In the ultrasonic oscillator unit 68 of the ultrasonic observation part 58 of Embodiment 1 illustrated in Figs. 1 to 4, the backing material layer 102 of the laminated body 86 has the stepped part 110. Thus, the space required for the wiring between the cable wiring part 88 and the cables 70 can be secured, and the plurality of cables 70 connected to the cable wiring part 88 can be kept from overlapping each other. However, the same effects can be exhibited even in a case where the stepwise stepped part 110 of the backing material layer 102 is formed in an inclined shape.

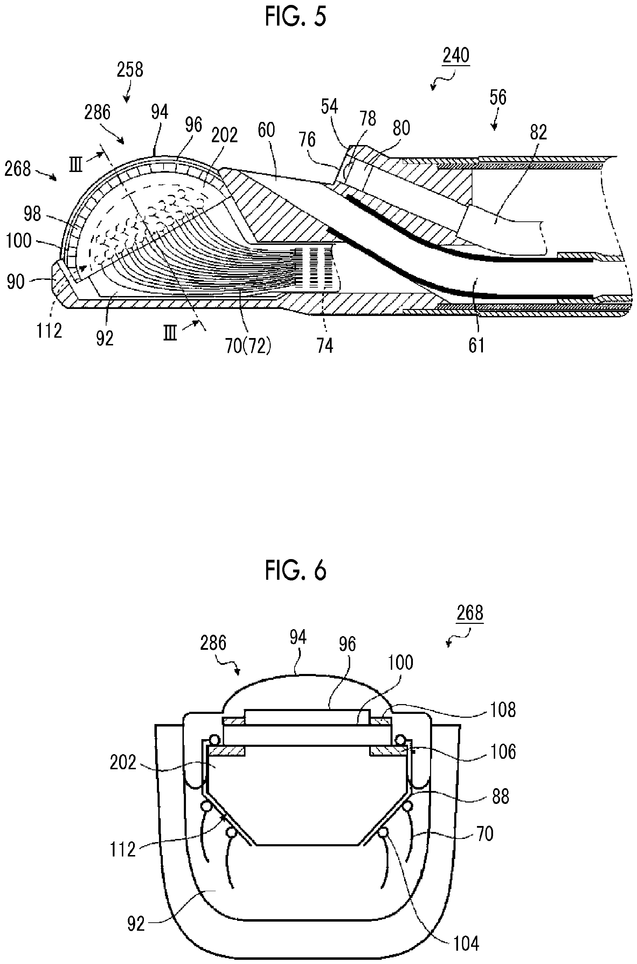

A cross-sectional view of the endoscope distal end part having the ultrasonic oscillator unit of the present embodiment (Embodiment 2) cut in the longitudinal direction thereof so as to pass through the center of the ultrasonic observation part in a width direction thereof is illustrated in Fig. 5. Additionally, Fig. 6 is a view of the endoscope distal end part taken along line III-III illustrated in Fig. 5 and seen from an arrow direction and is a cross-sectional view of an example of the ultrasonic observation part of the endoscope distal end part of the ultrasonic endoscope illustrated in Fig. 5. An ultrasonic observation part 258 of the endoscope distal end part 240 illustrated in Figs. 5 and 6 has the same structure as the ultrasonic observation part 58 of the ultrasonic endoscope 12 illustrated in Figs. 1 to 4 except for a backing material layer 202 of an ultrasonic oscillator unit 268 having no stepwise shape.

As illustrated in Fig. 6, the backing material layer 202 of a laminated body 286 has an inclined part 112 that becomes thinner in an inclined fashion toward the side opposite to the arrangement surface of the plurality of ultrasonic oscillators 98 constituting the ultrasonic oscillator array 100, on each side surface of the backing material layer 202 in the width direction thereof, in order to widely secure the space required for the wiring between the electrode part 106 of the ultrasonic oscillator array 100 and the cables 70 of the cable dispersion part 72.

Additionally, the cable wiring part 88 electrically connected to the electrode part 106 of the ultrasonic oscillator array 100 and the cables 70 is disposed along the inclined part 112 formed on each side surface of the backing material layer 202 in the width direction thereof.

The inclined part 112 formed on each side surface of the backing material layer 202 in the width direction thereof is provided so as to widely secure the space required for the wiring between the electrode part 106 of the ultrasonic oscillator array 100 and the cables 70 of the cable dispersion part 72 and such that the plurality of cables 70 electrically connected to the electrode part 106 do not overlap each other. Since the backing material layer 202 has a function of damping the ultrasonic waves propagated to the lower side of the ultrasonic oscillator array 100, it is preferable that the inclined part 112 is provided so as not to affect the output of the ultrasonic waves oscillated from the ultrasonic oscillator array 100, similarly to the stepped part 110 illustrated in Fig. 4. For this reason, it is preferable that the inclined part 112 is formed only in a region outside each side surface of the acoustic matching layer 96 in the width direction thereof on each side surface of the backing material layer 202 in the width direction thereof, in a region where the thickness from the surface on which the backing material layer 202 and the ultrasonic oscillator array 100 abut against each other is equal to or less than 3 mm. Additionally, the inclined part 112 may be formed in a region inside each side surface of the acoustic matching layer 96 in the width direction thereof on each side surface of the backing material layer 202 in the width direction thereof, in a region where the thickness from the surface on which the backing material layer 202 and the ultrasonic oscillator array 100 abut against each other exceeds 3 mm. In addition, the inclination of the inclined part 112 may be appropriately changed to such a degree that the height and the width become smaller toward the side opposite to the arrangement surface of the plurality of ultrasonic oscillators 98 that constitute the ultrasonic oscillator array 100 and the workability in a case where the cables 70 are wired to the cable wiring part 88 disposed along the inclined part 112 is not impaired, and the inclination may vary in the middle of the inclination of the inclined part 112.

As described above, by disposing the cable wiring part 88 electrically connected to the electrode part 106 of the ultrasonic oscillator array 100 and the cables 70 of the cable dispersion part 72 along the inclined part 112 formed in an inclined fashion toward the side opposite to the arrangement surface of the plurality of ultrasonic oscillators 98 that constitute the ultrasonic oscillator array 100 on each side surface of the backing material layer 202 of the laminated body 286 in the width direction thereof, a sufficient space for wiring the electrode part 106 and the cables 70 can be secured, and the plurality of wired cables 70 can be kept from overlapping each other.

Although the invention has been described above in detail, it is natural that the invention is not limited to the above embodiment, and various improvements and modifications may be made without departing from the scope of the invention, as defined by the appended claims.

Explanation of References

10: ultrasonic inspection system
12: ultrasonic endoscope
14: ultrasonic wave processor device
16: endoscope processor device
18: light source device
20: monitor
22: water supply tank
24: suction pump
26: insertion part
28: operating part
30: universal cord
32: air/water supply button
34: suction button
36: angle knob
38: treatment tool insertion port
40, 240: endoscope distal end part
42: bending part
44: flexible part
46: ultrasonic wave connector
48: endoscope connector
50: light source connector
52a, 52b: tube
54: inclined surface part

56: endoscope observation part
58, 258: ultrasonic observation part
60: treatment tool delivery port
61: treatment tool insertion channel
62: air/water supply nozzle
64: imaging unit
66: illumination unit
68, 268: ultrasonic oscillator unit
70: cable
72: cable dispersion part
74: cable covering part
76: observation window
78: objective lens
80: imaging element
82: signal cable
84: illumination window
86, 286: laminated body
88: cable wiring part
90: housing
92: filler layer
94: acoustic lens
96: acoustic matching layer
98: ultrasonic oscillator
100: ultrasonic oscillator array
102, 202: backing material layer
104: wiring pad row
106: electrode part
108: upper electrode part
110: stepped part
112: inclined part


**Claims**

1. An ultrasonic oscillator unit (68, 268) comprising:

an ultrasonic oscillator array (100) in which a plurality of ultrasonic oscillators (98) are arranged outward in a semicylindrical shape;
an electrode part (106) having a plurality of electrodes, each of two parts of the electrode part (106) being provided on a respective portion of the back surface of the ultrasonic oscillator array (100) opposite the arrangement surface of the plurality of ultrasonic oscillators (98) and being electrically connected to the plurality of ultrasonic oscillators, respectively, wherein each portion is adjacent a respective end surface side of the ultrasonic oscillator array (100) perpendicular to the arrangement surface;
a backing material layer (102, 202) that is disposed on the back surface of the ultrasonic oscillator array (100) serving as an inside with respect to said arrangement surface of the plurality of ultrasonic oscillators (98), wherein each of the two parts of the electrode part (106) is disposed within a gap between the backing material layer (102, 202) and the back surface at a corner of the backing material layer (102, 202) at a respective side thereof; and
a cable wiring part (88) in which a plurality of cables (70) are respectively disposed on a plurality of wiring lines electrically connected to the plurality of electrodes of the electrode part (106),
wherein the width of the backing material layer (102, 202) becomes smaller toward the side of the backing material layer opposite to said arrangement surface of the plurality of ultrasonic oscillators (98) such that (i) the backing material layer (102) has a stepwise stepped part (110) that becomes thinner toward a side opposite to the arrangement surface, on each side surface of the backing material layer (102) in the width direction thereof or (ii) the backing material layer (202) has an inclined part (112) that becomes thinner in an inclined fashion toward the side opposite to the arrangement surface, on each side surface of the backing material layer (202) in the width direction thereof, and
wherein the cable wiring part (88) is provided along the width of the backing material layer (102, 202) such that the cable wiring part (88) is disposed on side surfaces of the backing material layer (102, 202) in the width direction thereof.

2. The ultrasonic oscillator unit according to claim 1,
   wherein the backing material layer (102, 202) becomes thinner toward the side of the backing material layer (102, 202) opposite to said arrangement surface of the plurality of oscillators (98) in a region outside a side surface, in a width direction, of an acoustic matching layer (96) installed on an upper surface of the ultrasonic oscillator array (100).

3. The ultrasonic oscillator unit according to claim 1 or 2, further comprising:

   a flexible printed wiring board pasted to a side surface side of the ultrasonic oscillator array (100) and electrically connected to the plurality of electrodes of the electrode part (106),
   wherein the cable wiring part (88) has a plurality of cables (70) respectively disposed on a plurality of wiring lines electrically connected to the plurality of electrodes of the electrode part (106) via the flexible printed wiring board.

4. The ultrasonic oscillator unit according to any one of claims 1 to 3,
   wherein the backing material layer (102, 202) becomes thinner toward the side of the backing material layer (102, 202) opposite to said arrangement surface of the plurality of ultrasonic oscillators (98) in a region where a thickness from a surface abutting against the ultrasonic oscillator array (100) is equal to or less than 3 mm and in a region outside the side surface of the acoustic matching layer (96) in the width direction thereof.

5. The ultrasonic oscillator unit according to any one of claims 1 to 4,
   wherein the backing material layer (102, 202) becomes thinner toward the side of the backing material layer (102, 202) opposite to said arrangement surface of the plurality of ultrasonic oscillators (98) in a region where a thickness from a surface abutting against the ultrasonic oscillator array exceeds than 3 mm and in a region inside the side surface of the acoustic matching layer (96) in the width direction thereof.

6. The ultrasonic oscillator unit according to any one of claims 1 to 5, further comprising:

   a housing (90) that surrounds a portion from the side surface of the backing material layer (102, 202) in the width direction thereof to the cable wiring part (88) and a lower side of the backing material layer (102, 202); and
   a filler layer (92) that fills a gap between the backing material layer (102, 202) and the housing (96).

7. The ultrasonic oscillator unit according to claim 6,
   wherein, in a case where an acoustic impedance of the filler layer (92) is defined as Zp and an acoustic impedance of the backing material layer (102, 202) is defined as Zb, an acoustic impedance reflectivity Q of the filler layer (92) and the backing material layer (102, 202), which is expressed using the following Equation (1) is 50% or less,

$$Q = 100 \times |Zp - Zb|/(Zp + Zb) \qquad (1)$$

Here, the unit of the acoustic impedance Zp and Zb is $kg/m^2s$.

8. The ultrasonic oscillator unit according to claim 6 or 7,
   wherein the thermal conductivity of the filler layer (92) is equal to or more than 1.0 W/(m·K).

**Patentansprüche**

1. Ultraschalloszillatoreinheit (68, 268), umfassend:

   ein Ultraschalloszillatorarray (100), in welchem eine Mehrzahl von Ultraschalloszillatoren (98) in einer halbzylindrischen Form nach außen angeordnet sind;
   einen Elektrodenteil (106) mit einer Mehrzahl von Elektroden, wobei jeweils zwei Teile des Elektrodenteils (106) an einem jeweiligen Abschnitt auf der Rückfläche des Ultraschalloszillatorarrays (100) abgewandt von der Anordnungsfläche der mehreren Ultraschalloszillatoren (98) vorhanden und elektrisch mit den mehreren Ultraschalloszillatoren verbunden sind, wobei jeder Abschnitt einer jeweiligen Stirnflächenseite des Ultraschalloszillatorarrays (100) rechtwinklig zu der Anordnungsfläche eine Trägermaterialschicht (102, 202), die sich auf der Rückseite des Ultraschalloszillatorarrays (100) befindet und als eine Innenseite bezüglich der Anordnungsfläche der mehreren Ultraschalloszillatoren (98) dient, wobei jeder der zwei Teile des Elektrodenteils (106) innerhalb einer Lücke zwischen der Trägermaterialschicht (102, 202) und einer Rückfläche an einer Ecke der Trägermaterialschicht (102, 202) an einer jeweiligen Seite davon angeordnet ist; und

einen Kabelverdrahtungsteil (88), in welchem mehrere Kabel (70) an einer Mehrzahl von Verdrahtungsleitungen angeordnet ist, die elektrisch mit den mehreren Elektroden des Elektrodenteils (106) verbunden sind, wobei die Breite der Trägermaterialschicht (102, 202) in Richtung der Seite der Trägermaterialschicht abgewandt von der Anordnungsfläche der mehreren Ultraschalloszillatoren (98) in der Weise kleiner wird, dass (i) die Trägermaterialschicht (102) einen schrittweise abgestuften Teil (110) aufweist, der in Richtung der Seite abgewandt von der Anordnungsfläche dünner wird auf jeder Seitenfläche der Rückmaterialschicht (102) in deren Breitenrichtung, oder (ii) die Trägermaterialschicht (202) einen geneigten Teil (112) besitzt, der in einer geneigten Weise zu der der Anordnungsfläche abgewandten Seite hin an jeder Seitenfläche der Trägermaterialschicht (202) in deren Breitenrichtung dünner wird, und

wobei der Kabelverdrahtungsteil (88) entlang der Breite der Trägermaterialschicht (102, 202) derart angeordnet ist, dass der Kabelverdrahtungsteil (88) an Seitenflächen der Trägermaterialschicht (102, 202) in deren Breitenrichtung angeordnet ist.

2.  Ultraschalloszillatoreinheit nach Anspruch 1,
    bei der die Trägermaterialschicht (102, 202) in Richtung der Seite der Trägermaterialschicht (102, 202) abgewandt von der Anordnungsfläche der mehreren Oszillatoren (98) innerhalb einer Zone außerhalb einer Seitenfläche in einer Breitenrichtung einer akustischen Anpassschicht (96), die sich auf einer Oberseite des Ultraschalloszillatorarrays (100) befindet, dünner wird.

3.  Ultraschalloszillatoreinheit nach Anspruch 1 oder 2, weiterhin umfassend:

    eine flexible Schaltungsplatine, angeklebt an einer Seitenflächenseite des Ultraschalloszillatorarrays (100) und elektrisch mit den mehreren Elektroden des Elektrodenteils (106) verbunden,
    wobei der Kabelverdrahtungsteil (88) eine Mehrzahl von Kabeln (70) aufweist, die jeweils an einer Mehrzahl von Verdrahtungsleitungen angeordnet sind, die elektrisch mit den mehreren Elektroden des Elektrodenteils (106) über die flexible Schaltungsplatine verbunden sind.

4.  Ultraschalloszillatoreinheit nach einem der Ansprüche 1 bis 3,
    bei der die Trägermaterialschicht (102, 202) in Richtung der Seite der Trägermaterialschicht (102, 202) abgewandt von der Anordnungsfläche der mehreren Ultraschalloszillatoren (98) in einer Zone dünner wird, wo eine Dicke von einer Fläche, die gegen das Ultraschalloszillatorarray (100) anliegt, gleich oder kleiner als 3 mm ist, und in einer Zone außerhalb der Seitenfläche der akustischen Anpassschicht (96) in deren Breitenrichtung.

5.  Ultraschalloszillatoreinheit nach einem der Ansprüche 1 bis 4,
    bei der die Trägermaterialschicht (102, 202) in Richtung der Seite der Trägermaterialschicht (102, 202) abgewandt von der Anordnungsfläche der mehreren Ultraschalloszillatoren in einer Zone dünner wird, wo eine Dicke von einer Fläche, die gegen das Ultraschalloszillatorarray anschlägt, 2 mm übersteigt, und in einer Zone im Inneren der Seitenfläche der akustischen Anpassschicht (96) in deren Breitenrichtung.

6.  Ultraschalloszillatoreinheit nach einem der Ansprüche 1 bis 5, weiterhin umfassend:

    ein Gehäuse (90), welches einen Abschnitt von einer Seitenfläche der Trägermaterialschicht (102, 202) in deren Breitenrichtung bis zu dem Kabelverdrahtungsteil (88) und einer Unterseite der Trägermaterialschicht (102, 202) umgibt; und
    eine Füllschicht (92), die eine Lücke zwischen der Trägermaterialschicht (102, 202) und dem Gehäuse (96) ausfüllt.

7.  Ultraschalloszillatoreinheit nach Anspruch 6,
    bei der für den Fall, dass eine akustische Impedanz der Füllschicht (92) als Zp definiert ist und eine akustische Impedanz der Trägermaterialschicht (102, 202) als Zb definiert ist, eine akustische Impedanz-Reflektivität Q der Füllschicht (92) und der Trägermaterialschicht (102, 202) ausgedrückt durch die folgende Gleichung (1), 50% oder weniger beträgt,

$$Q = 100 \text{ x } \left| Zp - Zb \right| /(Zp + Zb) \qquad (1)$$

wobei die Einheit der akustischen Impedanz Zp und Zb die Einheit kg/m$^2$s hat.

**8.** Ultraschalloszillatoreinheit nach Anspruch 6 oder 7,
bei der die Wärmeleitfähigkeit der Füllschicht (92) gleich oder größer als 1,0 W/(m•K) ist.


**Revendications**

**1.** Unité à oscillateur ultrasonique (68, 268), comprenant :

un réseau d'oscillateurs ultrasoniques (100), où une pluralité d'oscillateurs ultrasoniques (98) sont agencés vers l'extérieur suivant une forme semi-cylindrique ;
une partie formant électrode (106), présentant une pluralité d'électrodes, chacune de deux parties de la partie formant électrode (106) étant respectivement prévue sur une portion respective de la surface arrière de l'agencement de la pluralité d'oscillateurs ultrasoniques (100) face à la surface d'agencement de la pluralité d'oscillateurs ultrasoniques (98) et étant respectivement connectée par voie électrique à la pluralité d'oscillateurs ultrasoniques, dans laquelle chaque portion est adjacente à un côté de surface d'extrémité respectif de l'agencement d'oscillateurs ultrasoniques (100) perpendiculairement à la surface d'agencement ;
une couche de matériau de support (102, 202), laquelle est disposée sur la surface arrière du réseau d'oscillateurs ultrasoniques (100) servant d'intérieur par rapport à ladite surface d'agencement de la pluralité d'oscillateurs ultrasoniques (98), dans laquelle chacune des deux parties de la partie formant électrode (106) est disposée dans un espace entre la couche de matériau de support (102, 202) et la surface arrière sur un coin de la couche de matériau de support (102, 202) sur un côté respectif de celle-ci, et
une partie de câblage par câbles (88), où une pluralité de câbles (70) sont respectivement disposés sur une pluralité de lignes de câblage connectées par voie électrique à la pluralité d'électrodes de la partie formant électrode (106) ;
dans laquelle la largeur de la couche de matériau de support (102, 202) diminue vers le côté de la couche de matériau de support face à ladite surface d'agencement de la pluralité d'oscillateurs ultrasoniques (98) de telle sorte que (i) la couche de matériau de support (102) présente une partie échelonnée (110) par échelons, laquelle s'amincit vers un côté face à la surface d'agencement, sur chaque surface latérale de la couche de matériau de support (102) dans la direction de largeur de celle-ci, ou (ii) la couche de matériau de support (202) présente une partie inclinée (112), laquelle s'amincit d'une manière inclinée vers le côté face à la surface d'agencement, sur chaque surface latérale de la couche de matériau de support (202) dans la direction de largeur de celle-ci, et dans laquelle la partie de câblage par câbles (88) est prévue le long de la largeur de la couche de matériau de support (102, 202) de telle sorte que la partie de câblage par câbles (88) est disposée sur des surfaces latérales de la couche de matériau de support (102, 202) dans la direction de largeur de celle-ci.

**2.** Unité à oscillateur ultrasonique selon la revendication 1,
dans laquelle la couche de matériau de support (102, 202) s'amincit vers le côté de la couche de matériau de support (102, 202) face à ladite surface d'agencement de la pluralité d'oscillateurs (98) dans une région à l'extérieur d'une surface latérale, dans une direction de largeur, d'une couche d'adaptation acoustique (96) installée sur une surface supérieure de l'agencement d'oscillateurs ultrasoniques (100).

**3.** Unité à oscillateur ultrasonique selon la revendication 1 ou 2, comprenant en outre :

une carte de câblage imprimée souple collée sur un côté de surface latérale de l'agencement d'oscillateurs ultrasoniques (100) et connectée par voie électrique à la pluralité d'électrodes de la partie formant électrode (106),
dans laquelle la partie de câblage par câbles (88) présente une pluralité de câbles (70) disposés respectivement sur une pluralité de lignes de câblage connectées par voie électrique à la pluralité d'électrodes de la partie formant électrode (106) via la carte de câblage imprimée souple.

**4.** Unité à oscillateur ultrasonique selon l'une quelconque des revendications 1 à 3,
dans laquelle la couche de matériau de support (102, 202) s'amincit vers le côté de la couche de matériau de support (102, 202) face à ladite surface d'agencement de la pluralité d'oscillateurs ultrasoniques (98) dans une région où une épaisseur à partir d'une surface aboutant contre l'agencement d'oscillateurs ultrasoniques (100) est inférieure ou égale à 3 mm et dans une région à l'extérieur de la surface latérale de la couche d'adaptation acoustique (96) dans la direction de largeur de celle-ci.

5. Unité à oscillateur ultrasonique selon l'une quelconque des revendications 1 à 4, dans laquelle la couche de matériau de support (102, 202) s'amincit vers le côté de la couche de matériau de support (102, 202) face à ladite surface d'agencement de la pluralité d'oscillateurs ultrasoniques (98) dans une région où une épaisseur à partir d'une surface aboutant contre l'agencement d'oscillateurs ultrasoniques est supérieure à 3 mm et dans une région à l'intérieur de la surface latérale de la couche d'adaptation acoustique (96) dans la direction de largeur de celle-ci.

6. Unité à oscillateur ultrasonique selon l'une quelconque des revendications 1 à 5, comprenant en outre :

   un logement (90), lequel entoure une portion allant de la surface latérale de de la couche de matériau de support (102, 202) dans la direction de largeur de celle-ci jusqu'à la partie de câblage par câbles (88) et un côté inférieur de la couche de matériau de support (102, 202), et
   une couche de charge (92), laquelle remplit un espace entre la couche de matériau de support (102, 202) et le logement (96).

7. Unité à oscillateur ultrasonique selon la revendication 6, dans laquelle dans un cas où une impédance acoustique de la couche de charge (92) est définie comme Zp et une impédance acoustique de la couche de matériau de support (102, 202) est définie comme Zb, une réflectivité d'impédance acoustique Q de la couche de charge (92) et de la couche de matériau de support (102, 202), laquelle est exprimée à l'aide de l'équation suivante (1), est inférieure ou égale à 50 %,

$$Q = 100 \text{ x } | Zp - Zb | / (Zp + Zb) \qquad (1)$$

l'unité de l'impédance acoustique Zp et Zb étant kg/m$^2$s.

8. Unité à oscillateur ultrasonique selon la revendication 6 ou 7, dans laquelle la conductivité thermique de la couche de charge (92) est supérieure ou égale à 1,0 W / (m · K).

# FIG. 1

EP 3 437 564 B1

## FIG. 2

## FIG. 3

## FIG. 4

## FIG. 5

## FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4445764 B
- JP 5399594 B
- JP 8004359 B
- JP H08004359 B
- JP 4980653 B
- JP 3802756 B
- US 2006103265 A1
- JP H0879895 A